# FASCICULE DE BREVET EUROPEEN

(11) **EP 2 670 463 B1**
(45) Date de publication et mention de la délivrance du brevet: **25.05.2016**
(21) Numéro de dépôt: 12704862.7
(22) Date de dépôt: 11.01.2012
(51) Int. Cl.: A61M 16/00, A61B 5/11, A61B 5/08, A61B 5/00

(54) **DISPOSITIF DE MESURE DE L'OBSERVANCE D'UN TRAITEMENT D'OXYGÉNOTHÉRAPIE À ACCÉLÉROMÈTRE TRIDIMENSIONNEL**
VORRICHTUNG ZUR MESSUNG DER ÜBEREINSTIMMUNG MIT EINER SAUERSTOFFTHERAPIE UNTER VERWENDUNG EINES DREIDIMENSIONALEN BESCHLEUNIGUNGSMESSERS
DEVICE FOR MEASURING COMPLIANCE WITH OXYGEN THERAPY USING A THREE-DIMENSIONAL ACCELEROMETER

(30) Priorité: 02.02.2011 FR 1150798
(43) Date de publication de la demande: 11.12.2013
(73) Titulaire: L'Air Liquide Société Anonyme pour l'Etude et l'Exploitation des Procédés Georges Claude, 75007 Paris (FR)
(72) Inventeur: WEBER, Claude, 91490 Milly La Foret (FR); BERNARD, Philippe, 60000 Goincourt (FR)
(74) Mandataire: Pittis, Olivier
(86) Numéro de dépôt international: PCT/FR2012/050071
(87) Numéro de publication internationale: WO 2012/104513

(56) Documents cités:
- EP-A1- 1 661 595
- FR-A1- 2 916 291
- FR-A1- 2 930 165
- US-A1- 2005 061 319

## Description

La présente invention concerne un dispositif de mesure de l'observance d'un traitement d'oxygénothérapie, ainsi qu'un système et un procédé de mesure correspondants.

Plus particulièrement, la présente invention concerne le domaine de l'observance d'un traitement médical d'oxygénothérapie, en particulier en soins à domicile.

De manière générale, connaître l'observance d'un traitement par un patient, c'est-à-dire mesurer le temps réel pendant lequel il suit son traitement est essentiel car cela permet de connaître l'efficacité du traitement et d'ajuster ainsi la prescription du traitement par le médecin traitant.

Le suivi de l'observance revêt une importance capitale dans le cas d'un traitement d'oxygénothérapie, généralement prescrit pour des patients atteints d'insuffisances respiratoires, en particulier de broncho-pneumopathie chronique obstructive, dite « BPCO ».

En effet, un traitement d'oxygénothérapie bien suivi peut conduire à une nette amélioration de la qualité de vie du patient et réduire les hospitalisations.

Cependant, les effets de l'oxygénothérapie sont négligeables, voire nuls, si le patient n'observe pas son traitement, par exemple si la prise d'oxygène s'effectue pendant une durée inférieure à quinze heures par jour.

Le document WO-A-2009/136101 décrit un dispositif destiné à la mise en oeuvre d'un système permettant la détection, chez un patient, de l'observance d'un traitement d'oxygénothérapie avec un apport d'oxygène. Ce dispositif comprend des moyens de détection du traitement d'oxygénothérapie pour évaluer des données relatives au traitement et des moyens d'enregistrement et de transmission de ces données.

Ce dispositif de télésurveillance utilisé par le patient à son domicile permet de mesurer quotidiennement le temps durant lequel le patient a suivi son traitement. Il constitue ainsi une aide au médecin traitant pour prédire les aggravations de la maladie et réduire le nombre et la durée des hospitalisations.

Cependant, ce dispositif ne prend pas en compte la variabilité des besoins du patient en oxygène, laquelle a une grande influence sur l'efficacité du traitement d'oxygénothérapie.

Le document FR-A-2916291 propose quant à lui un dispositif d'observance universel utilisable dans le cadre d'un traitement d'oxygénothérapie comprenant un dispositif de saisie de données du patient avec capteur de débit, d'activité respiratoire, moyens de mémorisation des données et une interface de communication permettant de communiquer avec une unité distante de traitement des données de manière à pouvoir visualiser des informations ou éditer des rapports. De manière optionnelle, ce dispositif peut comprend un capteur d'activité physique du patient.

Un autre dispositif d'observance utilisable dans le cadre d'un traitement d'oxygénothérapie est par ailleurs enseigné par le document EP-A-1661595.

La présente invention vise à proposer un dispositif de mesure de l'observance d'un traitement d'oxygénothérapie amélioré qui prenne en compte la variabilité des besoins en oxygène du patient et permette ainsi d'évaluer plus efficacement la performance du traitement d'oxygénothérapie.

De là, la présente invention concerne un dispositif de mesure de l'observance d'un traitement d'oxygénothérapie par un patient, comprenant un boîtier incorporant des moyens de détection du traitement d'oxygénothérapie, tel un ou plusieurs capteurs, pour évaluer des données relatives au traitement ; et des moyens de mesure d'un état d'activité physique du patient.

Selon un exemple, les moyens de mesure d'un état d'activité physique du patient comprennent :
- au moins un capteur de mouvement corporel du patient délivrant en sortie des données de déplacement corporel du patient dans l'espace ; et
- des moyens de traitement des données de déplacement corporel, tel un ou plusieurs microprocesseurs, pour fournir un état d'activité physique du patient, en particulier un état d'activité physique du patient choisi parmi un état de sommeil, un état de repos et un état d'activité, voire tout autre état d'activité physique.

Il est à noter qu'un état de sommeil n'indique pas forcément que le patient dort. Il peut aussi correspondre à un état d'inactivité du patient.

En effet, la prise en compte de l'état d'activité physique du patient par le dispositif selon l'invention, et plus précisément par notamment les moyens de mesure d'un état d'activité physique du patient, permet de corréler la consommation d'oxygène par le patient à cette activité et d'évaluer ainsi plus efficacement la performance du traitement d'oxygénothérapie.

En outre, l'intégration de ces différentes fonctions au niveau d'un même boîtier, c'est-à-dire d'un boîtier unique, permet une utilisation simplifiée pour le patient. De préférence, le boîtier est portatif et étanche.

Avantageusement, le boîtier du dispositif de l'invention comprend en outre des moyens de stockage des données relatives au traitement et de l'état d'activité physique du patient ; ou des moyens de transmission des données relatives au traitement et de l'état d'activité physique du patient vers un serveur distant ; ou les deux.

Un tel serveur distant peut être situé notamment dans le centre du prestataire par lequel le patient est suivi. Le médecin traitant dispose ainsi en temps réel des données d'observance du traitement suivi par le patient, mis à sa disposition par le prestataire.

De préférence, les moyens de stockage, telles une ou des mémoires de stockage d'informations, sont aptes à stocker également des données relatives au dispositif de mesure d'observance.

L'intégration de l'ensemble des moyens de mesure, de stockage et de transmission de données dans un seul boîtier permet d'avoir un dispositif facile d'utilisation par le patient et le personnel traitant.

Avantageusement, le capteur de mouvement corporel comprend un accéléromètre tridimensionnel. Un tel accéléromètre tridimensionnel présente l'avantage de pouvoir identifier de manière fiable les déplacements du patient et de pouvoir en déduire son état parmi les trois états précités quel que soit son positionnement par rapport au corps du patient. Il peut ainsi être porté indifféremment en bandoulière ou au niveau de la ceinture, par exemple, sans influencer l'estimation du type d'activité du patient.

L'utilisation d'un seul accéléromètre tridimensionnel suffit pour identifier de manière fiable l'état du patient parmi les trois états précités. Bien entendu, si besoin est, plusieurs accéléromètres peuvent être utilisés.

Avantageusement, le boîtier est étanche. Ainsi, le patient peut l'utiliser en permanence même quand il prend sa douche, ce qui renforce la pertinence des données relevées. En effet, les patients souffrant de BPCO prennent généralement leur douche en étant sous traitement d'oxygénothérapie, étant donné que l'apport d'oxygène est également recommandé lors de cette activité.

Par ailleurs, le boîtier est configuré pour être portable, c'est-à-dire portatif. De préférence, le poids du boîtier est inférieur à 200 g et se situe entre 20 et 100 g, notamment entre 25 et 80 g, par exemple entre environ 30 et 50 g.

Selon une réalisation préférée, le boîtier comprend une carte à circuit intégré dans laquelle sont prévus lesdits moyens de détection du traitement d'oxygénothérapie et lesdits moyens de mesure d'un état d'activité physique du patient. De préférence, ladite carte comprend au moins un microprocesseur commun aux moyens de détection du traitement d'oxygénothérapie et aux moyens de mesure d'un état d'activité physique du patient.

Avantageusement, le dispositif comprend une batterie unique d'alimentation électrique de la carte. Cette batterie permet au boîtier de fonctionner en totale autonomie, au moins pendant 1 an, de préférence encore pendant 2 ans.

Par ailleurs, le microprocesseur permet de mettre en oeuvre d'un côté un algorithme capable de transformer des mesures de pression en fréquences respiratoires du patient et d'en déduire la durée de traitement journalier et d'un autre côté un algorithme capable de déduire des données de déplacement corporel un état d'activité physique du patient.

En outre, le boîtier comprend au moins un passage de circulation de l'oxygène à l'intérieur, ledit passage étant isolé du reste du boîtier, c'est-à-dire qu'il est étanche au reste du boîtier.

Selon le cas, le dispositif de l'invention peut comprendre l'une ou plusieurs des caractéristiques suivantes :
- le ou les capteurs de pression et l'accéléromètre sont raccordés à un premier microprocesseur qui réalise un prétraitement des signaux de mesure issus du ou des capteurs de pression et de l'accéléromètre.
- un deuxième microprocesseur détermine, à partir des signaux prétraités par le premier microprocesseur, la présence ou non d'un traitement d'oxygénothérapie et commande l'enregistrement de données dans les moyens de stockage d'information.
- le deuxième microprocesseur commande l'enregistrement de données choisies parmi la durée de traitement d'oxygénothérapie, la fréquence respiratoire maximale et la fréquence respiratoire moyenne.
- le deuxième microprocesseur détermine, en outre, à partir des indicateurs des mouvements corporels du patient, un état d'activité du patient et commande l'enregistrement de l'état d'activité du patient dans les moyens de stockage d'information.

L'invention concerne également un système de mesure de l'observance d'un traitement d'oxygénothérapie suivi par un patient, comprenant le dispositif de mesure selon l'invention et des moyens de transmission d'informations entre le dispositif de mesure et un serveur distant.

A titre d'exemple, de tels moyens de transmission d'informations comprennent un ordinateur de bureau ou portable, un assistant numérique personnel (PDA), un modem ou tout autre dispositif de transmission d'information adéquat, par l'intermédiaire desquels les données relatives au traitement et l'état d'activité physique du patient sont transmis vers le serveur distant situé dans un centre de soins.

Avantageusement, le système de mesure comprend en outre une source d'oxygène ; et une canule délivrant de l'oxygène aux voies aériennes du patient, ledit dispositif de mesure étant configuré pour être raccordé à la source d'oxygène et à la canule.

Un exemple concerne également un procédé de mesure de l'observance d'un traitement d'oxygénothérapie par un patient, comprenant les étapes de :
- détection du traitement d'oxygénothérapie pour évaluer des données relatives au traitement; et
- mesure d'un état d'activité physique du patient.

Avantageusement, l'étape de mesure d'un état d'activité physique du patient comprend :
- une étape de mesure de données de déplacement corporel du patient dans l'espace ; et
- une étape de traitement des données de déplacement corporel pour fournir un état d'activité physique du patient choisi parmi un état de sommeil, un état de repos et un état d'activité.

On va maintenant décrire, de façon plus précise mais non limitative, un exemple de réalisation d'un dispositif et d'un système selon l'invention, en références aux Figures annexées parmi lesquelles :
- la Figure 1 est un schéma synoptique illustrant la structure d'un système de mesure de l'observance selon un mode de réalisation de l'invention ;
- la Figure 2 illustre le positionnement des axes d'un accéléromètre tridimensionnel dans le dispositif de la Figure 3;
- la Figure 3 est un schéma synoptique illustrant la structure d'un dispositif de mesure de l'observance selon un mode de réalisation de l'invention ;
- la Figure 4 est un schéma illustrant l'architecture électronique du dispositif des Figures 2 et 3; et
- la Figure 5 est un chronogramme illustrant le fonctionnement du procédé de mesure de l'observance selon un mode de réalisation de l'invention.

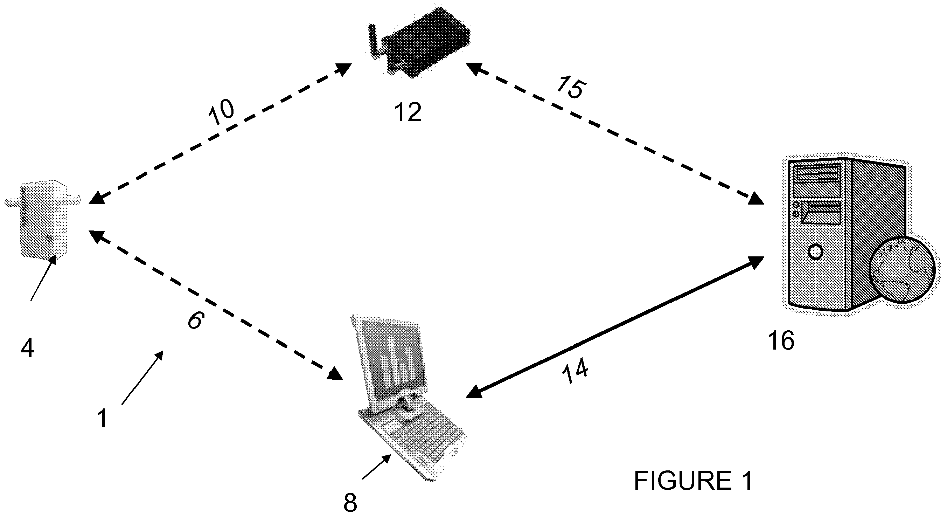

La Figure 1 illustre un système 1 de mesure de l'observance d'un traitement d'oxygénothérapie mettant en oeuvre un dispositif selon l'invention comprenant un boîtier 4.

Le boîtier 4 est portable ou portatif, c'est-à-dire configuré pour être porté par le patient à son domicile notamment, par exemple à la ceinture grâce à un système d'accroche adapté ou au cou grâce à un système d'accroche en pendentif.

Le boîtier 4 est raccordé, à travers une liaison de transmission d'informations 6, à des moyens d'enregistrement 8 aptes à enregistrer des données, par exemple un ordinateur personnel, un assistant numérique personnel (PDA) ou autre.

Le boîtier 4 est également raccordé, à travers une liaison de transmission d'informations 10, à des moyens de communication 12 formant passerelle de communication de données, par exemple un modem GSM ou GPRS.

Les liaisons de transmission 6 et 10 sont de préférence des liaisons radiofréquence (RF) dans les bandes de fréquence ISM («*Industrial Scientific Medical*») se situant entre 800 MHz et 5 GHz, de préférence entre 850 MHz et 3 GHz et de manière encore plus préférée de 868 Mhz ou 2,4 GHz.

En outre, les moyens d'enregistrement 8 sont raccordés à travers un réseau 14 de transmission de données, tel Internet, à un serveur distant 16 situé dans le centre du prestataire en charge du patient.

Les moyens de communication 12 sont également raccordés au serveur distant 16 à l'aide d'une liaison 15 de transmission d'informations, par exemple une liaison GSM ou GPRS, lorsque les moyens de communication 12 comprennent un modem GSM ou GPRS.

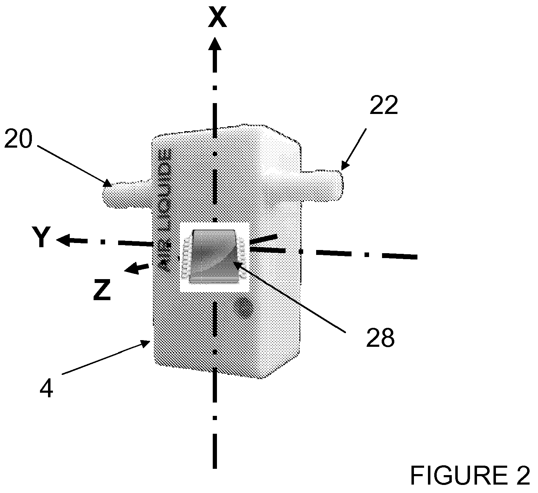

Ainsi que cela apparaît sur la Figure 2, le boîtier 4 comprend, aux extrémités du passage interne de gaz, deux embouts 20, 22 destinés à le raccorder d'une part à une source d'oxygène (non représentée) et d'autre part à une interface patient, par exemple une canule nasale (non représentée), voire un masque respiratoire.

Un passage de circulation d'oxygène est prévu dans le boîtier 4, ledit passage étant étanche par rapport au reste du boîtier, notamment des composants électroniques.

La source d'oxygène est choisie parmi les sources classiquement utilisées en oxygénothérapie, par exemple une bouteille d'oxygène comprimé ou un concentrateur d'oxygène ou encore un réservoir d'oxygène liquide. De préférence, le débit d'oxygène est compris entre 0,5 et 4 litres/minute. Bien entendu, ce débit est adapté à la prescription médicale.

On peut utiliser une canule standard, c'est-à-dire une canule qui n'est pas spécifiquement conçue pour une utilisation avec le dispositif de l'invention. Par exemple, il peut s'agir d'une canule selon la norme NF EN 13544-2 («*Respiratory Therapy équipment* - *Part 2 : Tubing and Connectons*») présentant un embout de connexion, comme par exemple une canule Intersurgical™, Salter Labs™ ou Octurno Medizintechnick™.

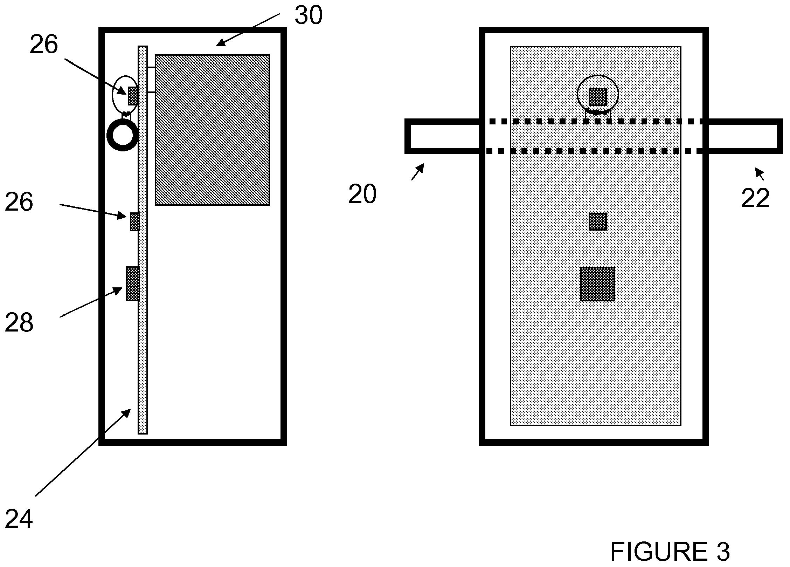

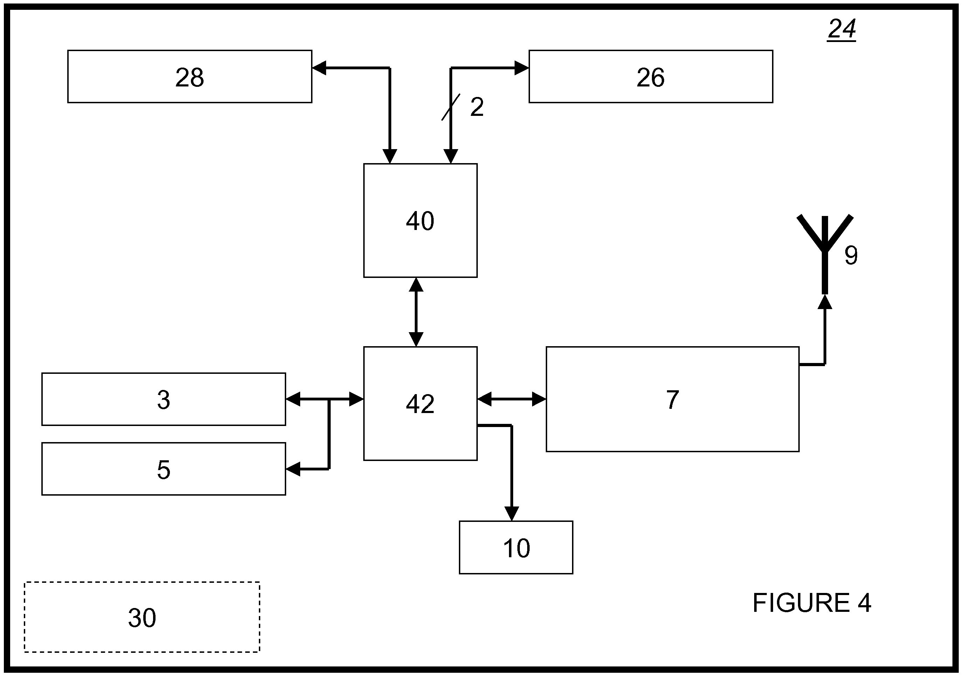

Le boîtier 4 comprend également une carte 24 à circuit intégré. Comme schématisé sur les Figures 3 et 4, cette carte 24 comprend deux capteurs de pression 26. Le premier capteur mesure la pression dans la canule due au débit d'oxygène et aux variations liées aux inspirations (correspondant à une dépression) et expirations (correspondant à une surpression) du patient dans la canule, et le deuxième capteur mesure la pression atmosphérique. La carte 24 est positionnée à cette fin vis-à-vis du passage de circulation de l'oxygène dans le passage de gaz du boîtier 4.

La carte 24 comprend également un accéléromètre tridimensionnel 28 dont les trois axes X, Y, Z sont orientés par rapport à la carte 24 de la manière représentée sur la Figure 2.

L'utilisation d'un accéléromètre tridimensionnel 28 permet d'avoir des mesures de mouvement corporel précises quelle que soit la position du boîtier 4. Ainsi, le patient est libre de porter ce boîtier 4 de la manière qu'il souhaite sans effet sur l'estimation du type d'activité du patient.

Le boîtier 4 comprend également une batterie 30 d'alimentation électrique de la carte 24. Cette batterie 30 possède une autonomie d'au minimum 1 an et si possible d'au moins 2 à 3 années.

Les capteurs de pression 26 et l'accéléromètre 28 sont raccordés à un microprocesseur 40 qui réalise un prétraitement des signaux de mesure issus des capteurs de pression 26 et de l'accéléromètre 28. Pour ce faire, on met en oeuvre un premier algorithme permettant de transformer des valeurs de pression en fréquences respiratoires du patient et un deuxième algorithme capable de déduire des accélérations mesurées, des indicateurs des mouvements corporels du patient.

Un deuxième microprocesseur 42 détermine, à partir des fréquences respiratoires du patient la présence ou non d'un traitement d'oxygénothérapie et commande l'enregistrement de la durée de traitement d'oxygénothérapie ainsi que de la fréquence respiratoire maximale et de la fréquence respiratoire moyenne dans des moyens de stockage d'information ou de données, comprenant une mémoire 3 FRAM et une mémoire 5 FLASH.

Le deuxième microprocesseur 42 détermine également, à partir des indicateurs des mouvements corporels du patient, un état d'activité du patient choisi parmi un état de sommeil ou d'inactivité, un état de repos et un état d'activité. Il commande également l'enregistrement de l'état d'activité du patient dans les moyens de stockage comprenant la mémoire 3 FRAM et la mémoire 5 FLASH.

Les mémoires 3 et 5 de stockage de données ont par exemple une capacité de 1 à 3 Mo, notamment 2 Mo, et une autonomie de 1 an minimum.

En outre, le deuxième microprocesseur 42 commande la transmission de la durée de traitement d'oxygénothérapie, la fréquence respiratoire maximale, la fréquence respiratoire moyenne et l'état d'activité du patient, à travers un module de communication radiofréquence 7 raccordé à une antenne 9, vers le serveur distant 16.

Les microprocesseurs 40, 42 et les mémoires 3,5 sont intégrées dans la carte 24.

De plus, le deuxième microprocesseur 42 est raccordée à une diode électroluminescente 10 (LED) qui constitue un moyen de communication avec le patient.

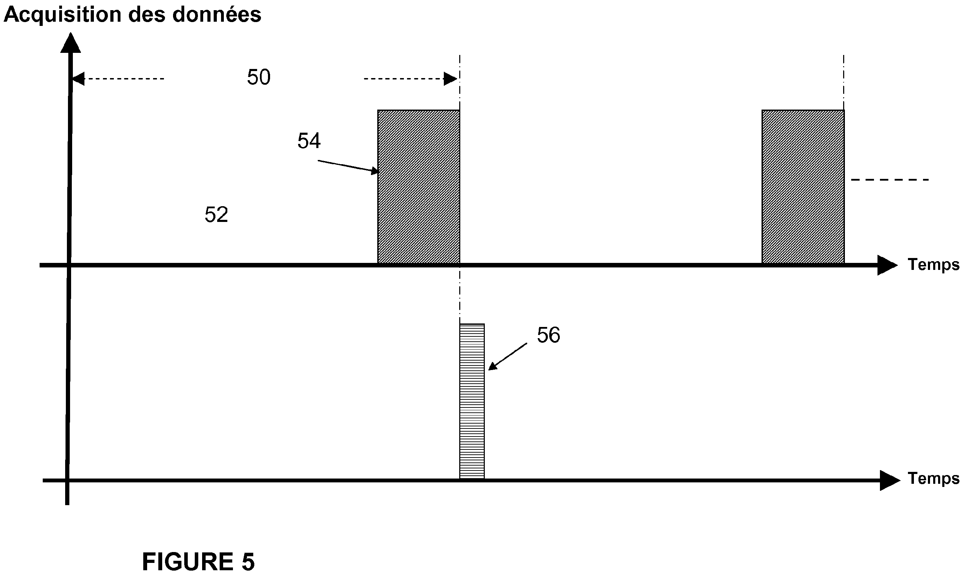

Le chronogramme de la Figure 5 illustre les différentes étapes du procédé de mesure de l'observance de l'invention.

Lors d'une étape A, des mesures de pression et d'accélération sont réalisées pendant des cycles de mesure 50 successifs. Chaque cycle de mesure 50 comprend une période 54 de mesure donnée, par exemple des périodes de plusieurs minutes à plusieurs heures, voire plusieurs jours, par les capteurs de mesure 26 et l'accéléromètre 28. La période 54 de mesure est suivie d'une période 56 de traitement des résultats de ces mesures par le microprocesseur 40. Pendant la période 52, aucune mesure n'est effectuée.

Lors d'une étape B, le microprocesseur 42 détermine, pendant la période 56, située immédiatement après le cycle 50, la durée de traitement d'oxygénothérapie, la fréquence respiratoire maximale, la fréquence respiratoire moyenne ainsi que l'état d'activité du patient, et commande le stockage de ces données dans les mémoires 44, 46.

Les cycles 50 se répètent dans le temps.

Le dispositif de mesure de l'observance d'un traitement d'oxygénothérapie selon l'invention particulièrement adapté à une utilisation de type soins à domicile, basé sur le suivi de l'état d'activité physique du patient, à savoir état de sommeil, état de repos ou état d'activité, voire tout autre état d'activité physique dans lequel peut se trouver le patient.

## Revendications

1. Dispositif de mesure de l'observance d'un traitement d'oxygénothérapie par un patient, comprenant un boîtier (4) contenant :
- des moyens de détection du traitement d'oxygénothérapie pour évaluer des données relatives au traitement ; et
- des moyens de mesure (28 ; 40, 42) d'un état d'activité physique du patient, comprenant au moins un capteur (28) de mouvement corporel du patient délivrant en sortie des données de déplacement corporel du patient dans l'espace, et des moyens de traitement (40, 42) des données de déplacement corporel pour fournir un état d'activité physique du patient,
- un ou plusieurs capteurs de pression (26), et
- des moyens de stockage (3, 5) des données relatives au traitement et à l'état d'activité physique du patient,
- le capteur de mouvement corporel comprenant comprend un accéléromètre tridimensionnel (28),
caractarisé en ce que
- le ou les capteurs de pression (26) et l'accéléromètre (28) sont raccordés à un premier microprocesseur (40) qui est configuré pour réaliser un prétraitement des signaux de mesure issus du ou des capteurs de pression (26) et de l'accéléromètre (28), et
- un deuxième microprocesseur (42) configuré pour déterminer, à partir des signaux prétraités par le premier microprocesseur (40), la présence ou non d'un traitement d'oxygénothérapie et configuré pour commander l'enregistrement de données dans les moyens de stockage (3, 5).

2. Dispositif selon la revendication 1, **caractérisé en ce que** le boîtier (4) comprend en outre des moyens de transmission (7, 9) des données relatives au traitement et de l'état d'activité physique du patient vers un serveur distant (16).

3. Dispositif selon l'une quelconque des revendications précédentes, **caractérisé en ce que** le boîtier (4) comprend une carte à circuit intégré (24) comprenant lesdits moyens de détection du traitement d'oxygénothérapie et lesdits moyens de mesure d'un état d'activité physique du patient.

4. Dispositif selon la revendication 3, **caractérisé en ce qu'**il comprend une batterie (30) unique d'alimentation de la carte (24).

5. Dispositif selon l'une quelconque des revendications 3 ou 4, **caractérisé en ce que** la carte (24) comprend au moins un microprocesseur (40, 42) commun aux moyens de détection du traitement d'oxygénothérapie et aux moyens de mesure d'un état d'activité physique du patient.

6. Dispositif selon l'une quelconque des revendications 1 ou 2, **caractérisé en ce qu'**au moins un microprocesseur (40, 42) est configuré pour coopérer avec les moyens de stockage (3, 5) de données et avec les moyens de transmission (7, 9) de données.

7. Dispositif selon l'une quelconque des revendications précédentes, **caractérisé en ce que** le boîtier (4) comprend au moins un passage de circulation de l'oxygène traversant ledit boîtier (4), ledit passage étant isolé du reste du boîtier (4) et comprenant deux embouts (20, 22) à ses extrémités.

8. Dispositif selon la revendication 1, **caractérisé en ce que** le deuxième microprocesseur (42) est configuré pour commander, l'enregistrement de données choisies parmi la durée de traitement d'oxygénothérapie, la fréquence respiratoire maximale et la fréquence respiratoire moyenne.

9. Dispositif selon l'une des revendications 1 ou 8, **caractérisé en ce que** le deuxième microprocesseur (42) est configuré pour déterminer, en outre, à partir des indicateurs des mouvements corporels du patient, un état d'activité du patient et commande l'enregistrement de l'état d'activité du patient dans les moyens de stockage d'information (3, 5).

10. Dispositif selon l'une des revendications précédentes, **caractérisé en ce que** les moyens de traitement (40, 42) des données de déplacement corporel permettent de fournir un état d'activité physique du patient choisi parmi un état de sommeil, un état de repos et un état d'activité.

11. Système de mesure de l'observance d'un traitement d'oxygénothérapie par un patient, comprenant un dispositif de mesure selon l'une quelconque des revendications 1 à 10 et des moyens de transmission d'informations (8,12) entre le dispositif de mesure et un serveur distant (16).

12. Système de mesure selon la revendication 11, **caractérisé en ce que** le dispositif de mesure est raccordé, d'une part, à une source d'oxygène et, d'autre part, à une interface délivrant de l'oxygène.

## Patentansprüche

1. Vorrichtung zur Messung der Einhaltung einer Sauerstofftherapiebehandlung durch einen Patienten, umfassend ein Gehäuse (4), das Folgendes enthält:
- Mittel zum Nachweis der Sauerstofftherapiebehandlung, um Daten mit Bezug auf die Behandlung zu bewerten; und
- Mittel zur Messung (28; 40, 42) eines Zustands der körperlichen Aktivität des Patienten, umfassend mindestens einen Sensor (28) der Körperbewegung des Patienten, der am Ausgang Daten der Körperbewegung des Patienten im Raum liefert, und Mittel zur Verarbeitung (40, 42) der Daten der Körperbewegung, um einen Zustand der körperlichen Aktivität des Patienten zu liefern,
- einen oder mehrere Drucksensoren (26), und
- Mittel zur Speicherung (3, 5) der Daten mit Bezug auf die Behandlung und den Zustand der körperlichen Aktivität des Patienten,
- wobei der Sensor der Körperbewegung einen dreidimensionalen Beschleunigungsmesser umfasst, **dadurch gekennzeichnet** (28), dass
- der oder die Drucksensoren (26) und der Beschleunigungsmesser (28) mit einem ersten Mikroprozessor (40) verbunden sind, der konfiguriert ist, um eine Vorverarbeitung der Messsignale durchzuführen, die von dem oder den Drucksensoren (26) und dem Beschleunigungsmesser (28) stammen, und
- einen zweiten Mikroprozessor (42), der konfiguriert ist, um auf der Grundlage der Signale, vorverarbeitet durch den ersten Mikroprozessor (40), die Anwesenheit oder Abwesenheit einer Sauerstofftherapiebehandlung zu bestimmen und konfiguriert ist, um die Aufzeichnung von Daten in die Mittel zur Speicherung (3, 5) anzuweisen.

2. Vorrichtung nach Anspruch 1, **dadurch gekennzeichnet, dass** das Gehäuse (4) außerdem Mittel zur Übertragung (7, 9) der Daten mit Bezug auf die Behandlung und den Zustand der körperlichen Aktivität des Patienten zu einem entfernten Server (16) umfasst.

3. Vorrichtung nach einem der vorhergehenden Ansprüche, **dadurch gekennzeichnet, dass** das Gehäuse (4) eine integrierte Leiterplatte (24), umfassend die Mittel zum Nachweis der Sauerstofftherapiebehandlung und die Mittel zur Messung eines Zustands der körperlichen Aktivität des Patienten, umfasst

4. Vorrichtung nach Anspruch 3, **dadurch gekennzeichnet, dass** sie eine einzige Batterie (30) zur Versorgung der Platte (24) umfasst.

5. Vorrichtung nach einem der Ansprüche 3 oder 4, **dadurch gekennzeichnet, dass** die Platte (24) mindestens einen Mikroprozessor (40, 42) umfasst, der den Mitteln zum Nachweis der Sauerstofftherapiebehandlung und den Mitteln zur Messung eines Zustands der körperlichen Aktivität des Patienten gemeinsam ist.

6. Vorrichtung nach einem der Ansprüche 1 oder 2, **dadurch gekennzeichnet, dass** mindestens ein Mikroprozessor (40, 42) konfiguriert ist, um mit den Mitteln zur Speicherung (3, 5) von Daten und mit den Mitteln zur Übertragung (7, 9) von Daten zusammenzuarbeiten.

7. Vorrichtung nach einem der vorhergehenden Ansprüche, **dadurch gekennzeichnet, dass** das Gehäuse (4) mindestens einen Durchgang zur Zirkulierung des Sauerstoffs umfasst, der das Gehäuse (4) durchquert, wobei der Durchgang vom Rest des Gehäuses (4) isoliert ist und zwei Spitzen (20, 22) an seinen Enden umfasst.

8. Vorrichtung nach Anspruch 1, **dadurch gekennzeichnet, dass** der zweite Mikroprozessor (42) konfiguriert ist, um die Aufzeichnung der Daten anzuweisen, ausgewählt aus der Dauer der Sauerstofftherapiebehandlung, der maximalen Atemfrequenz und der mittleren Atemfrequenz.

9. Vorrichtung nach einem der Ansprüche 1 oder 8, **dadurch gekennzeichnet, dass** der zweite Mikroprozessor (42) konfiguriert ist, um außerdem auf der Grundlage der Anzeigen der Körperbewegungen des Patienten einen Zustand der Aktivität des Patienten zu bestimmen, und die Aufzeichnung des Zustands der Aktivität des Patienten in den Mitteln zur Speicherung von Information (3, 5) anweist.

10. Vorrichtung nach einem der vorhergehenden Ansprüche, **dadurch gekennzeichnet, dass** die Mittel zur Verarbeitung (40, 42) der Daten der Körperbewegung ermöglichen, einen Zustand der körperlichen Aktivität des Patienten zu liefern, ausgewählt aus einem Zustand des Schlafs, einem Zustand der Ruhe und einem Zustand der Aktivität.

11. Vorrichtung zur Messung der Einhaltung einer Sauerstofftherapiebehandlung durch einen Patienten, umfassend eine Vorrichtung zur Messung nach einem der Ansprüche 1 bis 10 und Mittel zur Übertragung von Informationen (8, 12) zwischen der Vorrichtung zur Messung und einem entfernten Server (16).

12. System zur Messung nach Anspruch 11, **dadurch gekennzeichnet, dass** die Vorrichtung zur Messung einerseits mit einer Sauerstoffquelle und andererseits mit einer Schnittstelle verbunden ist, die Sauerstoff freisetzt.

## Claims

1. Device for measuring the observance of an oxygen-therapy treatment by a patient, comprising a housing (4) containing:
- means for detecting the oxygen-therapy treatment in order to evaluate data relating to the treatment; and
- means (28; 40, 42) for measuring a physical-activity state of the patient, comprising at least one sensor (28) for the body movement of the patient, delivering as an output data on the body movement of the patient in space, and means (40, 42) for processing the body-movement data in order to supply a physical-activity state of the patient,
- one or more pressure sensors (26), and
- means (3, 5) for storing data relating to the processing and to the physical-activity state of the patient,
- the body-movement sensor comprising a three-dimensional accelerometer (28),
**characterised in that**
- the pressure sensor or sensors (26) and the accelerometer (28) are connected to a first microprocessor (40) that is configured to carry out a pre-processing of the measurement signal issuing from the pressure sensor or sensors (26) and from the accelerometer (28), and
- a second microprocessor (42) configured so as to determine, from the signals pre-processed by the first microprocessor (40), the presence or not of an oxygen-therapy treatment and configured so as to control the recording of data in the storage means (3, 5).

2. Device according to claim 1, **characterised in that** the housing (4) further comprises means (7, 9) for transmitting the data relating to the processing and to the physical-activity state of the patient to a distant server (16).

3. Device according to any one of the preceding claims, **characterised in that** the housing (4) comprises an integrated circuit card (24) comprising said means for detecting the oxygen-therapy treatment and said means for measuring a physical-activity state of the patient.

4. Device according to claim 3, **characterised in that** it comprises a single battery (30) for supplying the card (24).

5. Device according to either one of claims 3 or 4, **characterised in that** the card (24) comprises at least one microprocessor (40, 42) common to the means for detecting the oxygen-therapy treatment and to the means for measuring a physical-activity state of the patient.

6. Device according to either one of claims 1 or 2, **characterised in that** at least one microprocessor (40, 42) is configured so as to cooperate with the data storage means (3, 5) and with the data transmission means (7, 9).

7. Device according to any one of the preceding claims, **characterised in that** the housing (4) comprises at least one passage for circulation of the oxygen passing through said housing (4), said passage being isolated from the rest of the housing (4) and comprising two connecting pieces (20, 22) at its ends.

8. Device according to claim 1, **characterised in that** the second microprocessor (42) is configured so as to control the recording of data chosen from the duration of the oxygen-therapy treatment, the maximum respiratory frequency and the mean respiratory frequency.

9. Device according to one of claims 1 or 8, **characterised in that** the second microprocessor (42) is configured so as to further determine, from the indicators of the body movements of the patient, a state of activity of the patient, and controls the recording of the state of activity of the patient in the information storage means (3, 5).

10. Device according to one of the preceding claims, **characterised in that** the means (40, 42) for processing the body-movement data make it possible to supply a physical-activity state of the patient chosen from a sleep state, a rest state and an activity state.

11. System for measuring the observance of an oxygen-therapy treatment by a patient, comprising a measurement device according to any one of claims 1 to 10 and means (8, 12) for transmitting information between the measurement device and a distant server (16).

12. Measurement system according to claim 11, **characterised in that** the measurement device is connected firstly to an oxygen source and secondly to an interface delivering oxygen.
